# EUROPEAN PATENT SPECIFICATION

(11) **EP 2 394 644 B1**
(45) Date of publication and mention of the grant of the patent: **09.07.2014**
(21) Application number: 11164658.4
(22) Date of filing: 03.05.2011
(51) Int. Cl.: A61K 9/24, A61K 31/495

(54) **Trimetazidine Formulation With Different Release Profiles**
Trimetazidin-Formulierung mit unterschiedlichen Freisetzungsprofilen
Formulation de trimetazidine avec différents profils de distribution

(30) Priority: 04.05.2010 TR 201003511
(43) Date of publication of application: 14.12.2011
(73) Proprietor: Sanovel Ilaç Sanayi Ve Ticaret Anonim Sirketi, 34460 Sariyer/Istanbul (TR)
(72) Inventor: CIFTER, Ümit, 34398 Istanbul (TR); TÜRKYILMAZ, Ali, 34398 Istanbul (TR); PEHLIVAN AKALIN, Nur, 34398 Istanbul (TR); YILDIRIM, Aylin, 34398 Istanbul (TR); ÖNER, Levent, Ankara (TR)
(74) Representative: Sevinç, Erkan

(56) References cited:
- WO-A1-99/33448
- WO-A1-2006/123073
- WO-A1-2007/054975
- WO-A2-2009/066315
- WO-A2-2010/084397
- KRISHNAIAH Y S R ET AL: "Three-layer guar gum matrix tablet formulations for oral controlled delivery of highly soluble trimetazidine dihydrochloride", JOURNAL OF CONTROLLED RELEASE, ELSEVIER, AMSTERDAM, NL, vol. 81, no. 1-2, 17 May 2002 (2002-05-17) , pages 45-56, XP004351102, ISSN: 0168-3659, DOI: DOI:10.1016/S0168-3659(02)00031-7
- UNKNOWN: 'Polyox' POLYOX WATER SOLUBLE RESINS, [Online] 01 March 2002, INTERNET, pages 1 - 24, XP055040540 Retrieved from the Internet: <URL:http://msdssearch.dow.com/PublishedLit eratureDOWCOM/dh_0031/0901b80380031a4a.pdf? filepath=polyox/pdfs/noreg/326-00001.pdf&fr omPage=GetDoc> [retrieved on 2012-10-10]

## Description

### Field of Invention

The present invention relates to formulations of trimetazidine or a pharmaceutically acceptable salt of trimetazidine. The present invention more particularly relates to a formulation of trimetazidine, providing immediate release and controlled release concomitantly.

### Background of Invention

Trimetazidine, with the chemical name 1-(2,3,4-trimethoxybenzyl)piperazine, has the following chemical structure of Formula I.

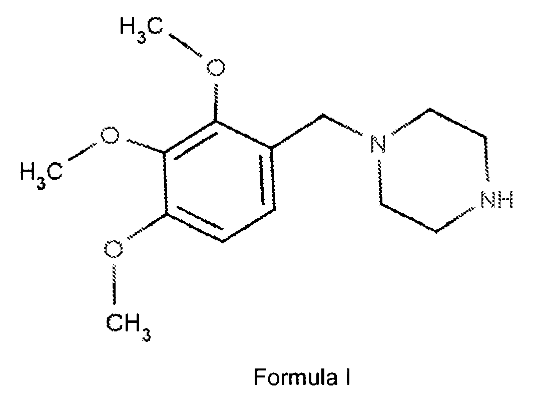

Trimetazidine is an anti-ischemic agent and used in the prophylaxis and treatment of angina pectoris and vertigo. It has considerably high solubility and is rapidly absorbed upon administration. Its half-life is 6 hours.

There are various patent applications available in relation to trimetazidine.
The application EP0533579 discloses trimetazidine derivatives and a process for obtaining the same.

The patent EP0673649 discloses a pharmaceutical composition providing prolonged release of trimetazidine or a pharmaceutically acceptable salt thereof. Prolonged release of trimetazidine is controlled by means of a reservoir system in this composition. In this reservoir system, a mixture of a water-insoluble polymer and a plasticizer is in the form of a film that coats tablets or minigranules containing trimetazidine or an addition salt thereof, together with a pharmaceutically acceptable acid. The plasticizing agent here is not hydrogenated oil.

The patent EP1108424 discloses a matrix object for prolonged release of trimetazidine or a pharmaceutically acceptable salt thereof, of which the prolonged release is controlled by using a polymer derived from cellulose, selected among hydroxypropyl methyl cellulose, hydroxyethyl cellulose, hydroxymethyl cellulose, methyl cellulose, hydroxypropyl cellulose.
The application EP1448173 discloses a process for preparing a "once a day" drug containing 60 mg trimetazidine dihydrochloride and having an pH-independent *in vitro* release, as well as a novel composition prepared by this process. Said composition is composed of uniformly-shaped and sized microbeads.

WO 2009/066315 discloses sustained release compositions of trimetazidine.

Various formulations have been developed with trimetazidine. Reviewed generally, it can be concluded that there are various conventional tablets and controlled-release formulations of trimetazidine molecule. The posology of such formulations is in the form of administering 3 conventional products or 2 controlled-release products per day. "Once a day" administration of doses gains high importance and need with respect to patients. The application EP 1 448 173 discloses a controlled-release tablet containing 60 mg trimetazidine dihydrochloride and claims to treat patients, needing such treatment, by administering one dose thereof daily. A certain period of time is required, however, to the onset of the effect of said formulation. This is not desirable for the patient. A quick onset of the treatment process directly affects patient life quality.

Trimetazidine dihydrochloride is highly-soluble in water. It is therefore quite difficult to develop a formulation of highly-soluble trimetazidine dihydrochloride with release profiles of desired quality. For instance, such release profiles may show fluctuations. On the other hand, polymers providing proper release profiles impose problems, such as sticking to punches and other equipment during production, due to their viscosities.

Considering the aforesaid problems, it is obvious that a novelty is needed in both providing production convenience in the art of "once a day" dose formulations containing trimetazidine dihydrochloride, and ensuring convenient release profiles for the same.

### Object and Brief Description of Invention

The present invention provides a trimetazidine formulation, eliminating all aforesaid problems and brining additional advantages to the relevant prior art.
Accordingly, the main object of the present invention is to provide a trimetazidine formulation administered orally once or twice per day.

Another object of the present invention is to reduce the treatment onset time, while said formulation provides a 24-hour effect.

A further object of the present invention is to prevent the production items from sticking to contact surfaces of machines and equipment.

A solid oral pharmaceutical formulation comprising trimetazidine or a pharmaceutically acceptable salt or polymorph of trimetazidine and having multiple layers has been developed to carry out all objects, referred to above and to emerge from the following detailed description.

According to a preferred embodiment of the present invention, said novelty is characterized in that one layer of said formulation provides controlled release, whereas the other layer provides immediate release.

According to a preferred embodiment of the present invention, said formulation comprises one or more excipients.

According to an embodiment of the present invention, said formulation comprises at least one intermediate layer.

According to a preferred embodiment of the present invention, said trimetazidine is trimetazidine dihydrochloride.

According to a preferred embodiment of the present invention, the amount of trimetazidine dihydrochloride in said immediate-release layer is not more than 25% and is preferably 10% by weight of the total amount of trimetazidine dihydrochloride present in the tablet.

According to the present invention, the controlled-release providing agent is polyethylene oxide. Said controlled-release agent is used in the outer granule phase.

According to a preferred embodiment of the present invention, the ratio of trimetazidine dihydrochloride in said controlled-release layer to polyethylene oxide is between 0.05 to 10, preferably 0.1 to 5, and more preferably 0.2 to 0.8.

According to a preferred embodiment of the present invention, said excipients comprise at least one or a mixture of binders, diluents, disintegrants, glidants, and lubricants.

According to a preferred embodiment of the present invention, said binder comprises at least one or a mixture of cellulose derivatives, such as polyvinylpyrrolidone (povidone), hydroxypropyl methyl cellulose, hydroxypropyl cellulose, carboxymethyl cellulose, methyl cellulose, ethyl cellulose; and gelatin; but is preferably polyvinylpyrrolidone.

According to a preferred embodiment of the present invention, said diluents comprise at least one or a mixture of lactose, starch, mannitol, calcium hydrogen phosphate dihydrate, dicalcium hydrogen phosphate anhydrate, calcium phosphate trihydrate, silicium dioxide and glucose; but is preferably selected from calcium hydrogen phosphate dihydrate and dicalcium hydrogen phosphate anhydrate.

According to a preferred embodiment of the present invention, said glidants comprise at least one or a mixture of colloidal silicone dioxide, talk, aluminum silicate, magnesium silicate; but is preferably colloidal silicone dioxide.

According to a preferred embodiment of the present invention, said formulation comprises magnesium stearate as a lubricant.

Another preferred embodiment according to the present invention consist of
a. immediate release layer
   a. trimetazidine dihydrochloride at 0.1 to 10% by weight,
   b. dicalcium hydrogen phosphate anhydrate at 5 to 90% by weight,
   c. colloidal silicone dioxide at 0.1 to 5% by weight,
   d. magnesium stearate at 0.1 to 5% by weight,
   e. red iron oxide at 0.01 to 5% by weight,
b. controlled release layer
   a. trimetazidine dihydrochloride at 2.5 to 50% by weight,
   b. polyethylene oxide at 2 to 90% by weight,
   c. polyvinylpyrrolidone at 0.1 to 20% by weight,
   d. calcium hydrogen phosphate dihydrate at 5 to 90% by weight,
   e. colloidal silicone dioxide at 0.1 to 5% by weight,
   f. magnesium stearate at 0.1 to 5% by weight.

A further preferred embodiment according to the present invention provides a method for preparing a pharmaceutical formulation, this method comprising the steps of
a. sieving and then mixing trimetazidine dihydrochloride, polyvinylpyrrolidone and calcium hydrogen phosphate dihydrate in a high-shear mixer,
b. granulating the mixture from above with sufficient amount of water,
c. sieving the wet granules formed, then drying the same and sieving back the dried granules,
d. adding polyethylene oxide and colloidal silicone dioxide into the granules obtained and mixing the latter,
e. sieving magnesium stearate and mixing it into the resultant mixture to obtain the controlled-release phase,
f. sieving and mixing together trimetazidine dihydrochloride, dicalcium hydrogen phosphate anhydrate, colloidal silicone dioxide and red iron oxide,
g. sieving magnesium stearate and mixing it into the resultant mixture to obtain the immediate-release phase,
h. compacting the controlled-release phase and immediate-release phase to provide a bilayer tablet.

### Detailed Description of Invention

### Example 01

| **Trimetazidine MR 35 mg Tablet Unit Formula (mg)** | | |
|---|---|---|
| | **Immediate-release phase (10%)** | **Controlled-release phase (90%)** |
| | | **Inner Phase** |
| Trimetazidine dihydrochloride | 3.5 | 31.5 |
| PVP K-90 F | - | 3.0 |
| Calcium hydrogen phosphate dihydrate | - | 50.0 |

| | | **Outer Phase** |
|---|---|---|
| Polyethylene oxide WSR 303 | - | 90.0 |
| Dicalcium hydrogen phosphate anhydrate | 37.5 | - |
| Magnesium stearate | 0.4 | 1.8 |
| Colloidal silicone dioxide | 0.2 | 0.9 |
| Red iron oxide | 0.2 | - |

### Producing the Controlled-release phase

Trimetazidine dihydrochloride, polyvinylpyrrolidone and calcium hydrogen phosphate dihydrate are sieved and mixed in a high-shear mixer. The resulting mixture is then granulated with an adequate amount of water. Wet granules formed are sieved, then dried and the dried granules are sieved back. Polyethylene oxide and colloidal silicone dioxide are added into and mixed together with the granules obtained. Polyethylene oxide providing controlled release is used in the outer granule phase. When polyethylene oxide is included into wet granules, it leads to problems of sticking over the machinery and equipment surfaces. Thus, polyethylene oxide is used in the outer phase to prevent this problem so that a substantial probable problem is avoided. Magnesium stearate is added into and mixed with this mixture to produce the controlled-release phase.

### Producing the Immediate-release phase

Trimetazidine dihydrochloride, dicalcium hydrogen phosphate anhydrate, colloidal silicone dioxide and red iron oxide are mixed in a separate kettle. To this mixture obtained is added sieved magnesium stearate to directly give the immediate-release phase. The controlled-release phase and the immediate-release phase are compacted to provide a bilayer tablet. The following table provides comparative data of dissolution profiles of Vastarel MR and the formulation coded 01C10 developed by us.

| **Time (minute)** | **Vastarel 0.1 N HCl** | **01C10 0.1 N HCl** |
|---|---|---|
| 0 | 0 | 0 |
| 15 | 16 | 19 |
| 30 | 24 | 29 |
| 45 | 30 | 37 |
| 60 | 36 | 44 |
| 120 | 53 | 65 |
| 180 | 67 | 80 |
| 240 | 79 | 88 |
| 300 | 87 | 92 |
| 360 | 93 | 94 |
| 420 | 99 | 99 |
| 480 | 100 | 99 |

### Example 02 (Reference)

| **Trimetazidine MR 35 mg Tablet Unit Formula (mg)** | | |
|---|---|---|
| | **Immediate-release phase (10%)** | **Controlled-release phase (90%)** |
| | | **Inner Phase** |
| Trimetazidine dihydrochloride | 3.5 | 31.5 |
| PVP K-90 F | - | 3.0 |
| Calcium hydrogen phosphate dihydrate | - | 70.0 |

| | | **Outer Phase** |
|---|---|---|
| Glyceryl behenate | - | 70.0 |
| Dicalcium hydrogen phosphate anhydrate | 37.5 | - |
| Magnesium stearate | 0.4 | 1.8 |
| Colloidal silicone dioxide | 0.2 | 0.9 |
| Red iron oxide | 0.2 | - |

Generally, when some controlled release agents are included into wet granules, it leads to problems of sticking over the machinery and equipment surfaces. Controlled release agents are used in the outer phase so that said agents prevent problems of sticking over the machinery and equipment. According to a preferred embodiment of the present invention controlled-release providing agent comprises at least one or a mixture of polymethacrylate, glyceryl behenate, polyvinylpyrrolidone (povidone), cross-linked polyvinylpyrrolidone, hydroxypropyl methyl cellulose (HPMC), hydroxypropyl cellulose (HPC), carboxymethyl cellulose (CMC), methyl cellulose (MC), ethyl cellulose (EC) and other cellulose derivatives, polyethylene oxide and gelatin; said controlled-release agent is preferably selected from glyceryl behenate, hydroxypropyl methyl cellulose (HPMC), hydroxypropyl cellulose (HPC), carboxymethyl cellulose (CMC), methyl cellulose (MC), ethyl cellulose (EC) and other cellulose derivatives, polyethylene oxide and gelatin; but said controlled-release agent is more preferably polyethylene oxide.

These studies conducted at 0.1 N HCl show that the 01 C01 coded product according to the present invention dissolves more, particularly during the first 3 hours. Thus, the time for the treatment onset is shortened.

This formulation is embodied for 35 and 70 mg trimetazidine tablets. 35 mg and 70 mg tablets are developed to provide drug activity up to 24 hours.

This invention has surprisingly provided a bilayer tablet formulation containing trimetazidine dihydrochloride, which does not stick to machinery during production and shows desired release profiles. The amounts of immediate-release and controlled-release phases in said bilayer tablet are such determined that 35 and 70 mg formulations are obtained of convenient and desired quality, which provide drug release up to 24 hours. The amount of trimetazidine dihydrochloride in said immediate-release layer is not more than 25% and is preferably 10% by weight of the total amount of trimetazidine dihydrochloride present in the tablet.

The formulation developed is used in treating angina pectoris, chorioretinal vascular disorders, tinnitus, vertigo, and meniere's syndrome.

Although the tablet obtained is bilayered, it is alternatively possible to design said tablet with more layers as well. It is possible to place one or more intermediate layers between the layers that contain the active agent in order to combine the layers, such intermediate layers possibly including an active agent or not including an active agent, and providing immediate release or controlled release. Whilst both layers including the active agent may provide controlled release or immediate release, at least one thereof may provide immediate release while the others provide controlled, prolonged, and retarded release.

It is further possible to use the following additional auxiliaries in the formulation.

Suitable binders include, but are not restricted to, at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to, at least one or a mixture of colloidal silicone dioxide, talk, and aluminum silicate.

Suitable lubricants include, but are not restricted to, at least one or a mixture of sodium stearil fumarat, magnesium stearate, polyethylene glycol, stearic acid, metal stearates, boric acid, sodium chloride benzoate and acetate, sodium or magnesium lauryl sulfate.

Suitable disintegrants include, but are not restricted to, at least one or a mixture of sodium starch glycolate, croscarmellose sodium, crospovidone, sodium alginate, gums, starch, and magnesium aluminum silicate.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, and magnesium lauryl sulfate.

Suitable coating agents include, but are not restricted to hydroxypropyl methyl cellulose, polyethylene glycol, polyvinylpyrrolidone, polyvinylpyrrolidone-vinyl acetate copolymer (PVP-VA), polyvinyl alcohol and other polymers, and all kinds of OpadryTM, as well as pigments, dyes, titanium dioxide and iron oxide, talk.

## Claims

1. A multilayered solid oral pharmaceutical formulation of trimetazidine or a pharmaceutically acceptable salt or polymorph of trimetazidine, **characterized in that** one layer of said formulation providing controlled release comprises a controlled-release providing agent which is polyethylene oxide in an outer granule phase, while the other layer provides immediate release.

2. The pharmaceutical formulation according to Claim 1, further comprising at least one or more excipients.

3. The pharmaceutical formulation according to any of the preceding claims, comprising preferably at least one intermediate layer.

4. The pharmaceutical formulation according to any of the preceding claims, wherein the amount by weight of trimetazidine dihydrochloride in said immediate-release layer is not more than 25% and is preferably 10% by weight of the total amount of trimetazidine dihydrochloride present in the tablet.

5. The pharmaceutical formulation according to any of the preceding claims, wherein the ratio of trimetazidine dihydrochloride in said controlled-release layer to polyethylene oxide is between 0.05 to 10, preferably 0.1 to 5, and more preferably 0.2 to 0.8.

6. The pharmaceutical formulation according to any of the preceding claims, wherein said excipient comprises at least one or a mixture of binders, diluents, disintegrants, glidants, and lubricants.

7. The pharmaceutical formulation according to claim 7, **characterized in that** said binder contains at least one or a mixture of polyvinylpyrrolidone (povidone), hydroxypropyl methyl cellulose, hydroxypropyl cellulose, carboxymethyl cellulose, methyl cellulose, ethyl cellulose and other cellulose derivatives, and gelatin; said binder being preferably polyvinylpyrrolidone.

8. The pharmaceutical formulation according to claim 7, **characterized in that** said diluent contains at least one or a mixture of lactose, starch, mannitol, calcium hydrogen phosphate dihydrate, dicalcium hydrogen phosphate anhydrate, calcium phosphate trihydrate, silicium dioxide and glucose; said diluent being preferably selected from calcium hydrogen phosphate dihydrate and dicalcium hydrogen phosphate anhydrate.

9. The pharmaceutical formulation according to claim 7, **characterized in that** said glidant comprises at least one or a mixture of colloidal silicone dioxide, talk, aluminum silicate, and magnesium silicate, said glidant being preferably colloidal silicone dioxide.

10. The pharmaceutical formulation according to claim 7, wherein the lubricant used comprises magnesium stearate.

11. The pharmaceutical formulation according to any of the preceding claims, consisting of
a. immediate-release layer
a. trimetazidine dihydrochloride at 0.1 to 10% by weight,
b. dicalcium hydrogen phosphate anhydrate at 5 to 90% by weight,
c. colloidal silicone dioxide at 0.1 to 5% by weight,
d. magnesium stearate at 0.1 to 5% by weight,
e. red iron oxide at 0.01 to 5% by weight;
b. controlled-release layer
a. trimetazidine dihydrochloride at 2.5 to 50% by weight,
b. polyethylene oxide at 2 to 90% by weight,
c. polyvinylpyrrolidone at 0.1 to 20% by weight,
d. calcium hydrogen phosphate dihydrate at 5 to 90% by weight,
e. colloidal silicone dioxide at 0.1 to 5% by weight,
f. magnesium stearate at 0.1 to 5% by weight.

12. A method for preparing a pharmaceutical formulation according to any of the preceding claims, comprising the steps of
a. sieving and then mixing trimetazidine dihydrochloride, polyvinylpyrrolidone and calcium hydrogen phosphate dihydrate in a high-shear mixer,
b. granulating the mixture from above with sufficient amount of water,
c. sieving the wet granules formed, then drying the same and sieving back the dried granules,
d. adding polyethylene oxide and colloidal silicone dioxide into the granules obtained and mixing the latter,
e. sieving magnesium stearate and mixing it into the resultant mixture to obtain the controlled-release phase,
f. sieving and mixing together trimetazidine dihydrochloride, dicalcium hydrogen phosphate anhydrate, colloidal dioxide and red iron oxide,
g. sieving magnesium stearate and mixing it into the resultant mixture to obtain the immediate-release phase,
h. compacting the controlled-release phase and immediate-release phase to provide a bilayer tablet.

13. Use of a pharmaceutical formulation as claimed in any of the preceding claims in producing a medicament for use in the treatment of angina pectoris, chorioretinal vascular disorders, tinnitus, vertigo, and meniere's syndrome.

## Patentansprüche

1. Mehrschichtige feste orale pharmazeutische Formulierung aus Trimetazidin oder einem pharmazeutisch akzeptablen Salz oder einer polymorphen Form von Trimetazidin, **dadurch gekennzeichnet, dass** eine die verzögerte Wirkstofffreisetzung bewirkende Schicht dieser Formulierung einen Wirkstoff zur Bewirkung der verzögerten Wirkstofffreisetzung umfasst, bei dem es sich um Polyethylenoxid in einer äußeren granulären Phase handelt, während die andere Schicht eine sofortige Wirkstofffreisetzung vermittelt.

2. Pharmazeutische Formulierung nach Anspruch 1, weiterhin umfassend mindestens einen oder mehrere Hilfsstoffe.

3. Pharmazeutische Formulierung nach einem der vorhergehenden Ansprüche, vorzugsweise umfassend mindestens eine Zwischenschicht.

4. Pharmazeutische Formulierung nach einem der vorhergehenden Ansprüche, bei der die Gewichtsmenge an Trimetazidindihydrochlorid in der Schicht mit sofortiger Wirkstofffreisetzung nicht mehr als 25 % beträgt und vorzugsweise bei 10 Gew.-% der Gesamtmenge des in der Tablette enthaltenen Trimetazidindihydrochlorids liegt.

5. Pharmazeutische Formulierung nach einem der vorhergehenden Ansprüche, bei der das Verhältnis von Trimetazidindihydrochlorid in der Schicht mit verzögerter Wirkstofffreisetzung zu Polyethylenoxid 0,05 bis 10, vorzugsweise 0,1 bis 5, und noch bevorzugter 0,2 bis 0,8 beträgt.

6. Pharmazeutische Formulierung nach einem der vorhergehenden Ansprüche, bei der der Hilfsstoff mindestens einen oder eine Mischung folgender Stoffe umfasst: Bindemittel, Verdünnungsmittel, Sprengmittel, Fließreguliermittel und Schmiermittel.

7. Pharmazeutische Formulierung nach Anspruch 7, **dadurch gekennzeichnet, dass** das Bindemittel mindestens einen oder eine Mischung folgender Stoffe enthält: Polyvinylpyrrolidon (Povidon), Hydroxypropylmethylcellulose, Hydroxypropylcellulose, Carboxymethylcellulose, Methylcellulose, Ethylcellulose und andere Cellulosederivate und Gelatine; wobei das Bindemittel vorzugsweise Polyvinylpyrrolidon ist.

8. Pharmazeutische Formulierung nach Anspruch 7, **dadurch gekennzeichnet, dass** das Verdünnungsmittel mindestens einen oder eine Mischung folgender Stoffe enthält: Lactose, Stärke, Mannitol, Calciumhydrogenphosphat-Dihydrat, Dicalciumhydrogenphosphat-Anhydrat, Calciumphosphat-Trihydrat, Siliziumdioxid und Glukose; wobei das Verdünnungsmittel vorzugsweise aus Calciumhydrogenphosphat-Dihydrat und Dicalciumhydrogenphosphat-Anhydrat ausgewählt ist.

9. Pharmazeutische Formulierung nach Anspruch 7, **dadurch gekennzeichnet, dass** das Fließreguliermittel mindestens einen oder eine Mischung folgender Stoffe umfasst: kolloidales Siliziumdioxid, Talk, Aluminiumsilikat und Magnesiumsilikat, wobei das Fließreguliermittel vorzugsweise kolloidales Siliziumdioxid ist.

10. Pharmazeutische Formulierung nach Anspruch 7, bei der das verwendete Schmiermittel Magnesiumstearat umfasst.

11. Pharmazeutische Formulierung nach einem der vorhergehenden Ansprüche, bestehend aus:
a. einer Schicht mit sofortiger Wirkstofffreisetzung aus:
a) Trimetazidindihydrochlorid, zu 0,1 bis 10 Gew.-%,
b) Dicalciumhydrogenphosphat-Anhydrat, zu 5 bis 90 Gew.-%,
c) kolloidalem Siliziumdioxid, zu 0,1 bis 5 Gew.-%,
d) Magnesiumstearat, zu 0,1 bis 5 Gew.-%,
e) rotem Eisenoxid, zu 0,01 bis 5 Gew.-%;
b. einer Schicht mit verzögerter Wirkstofffreisetzung aus:
a) Trimetazidindihydrochlorid, zu 2,5 bis 50 Gew.-%,
b) Polyethylenoxid, zu 2 bis 90 Gew.-%,
c) Polyvinylpyrrolidon zu 0,1 bis 20 Gew.-%,
d) Calciumhydrogenphosphat-Dihydrat, zu 5 bis 90 Gew.-%,
e) kolloidalem Siliziumdioxid, zu 0,1 bis 5 Gew.-%;
f) Magnesiumstearat, zu 0,1 bis 5 Gew.-%.

12. Verfahren zur Herstellung einer pharmazeutischen Formulierung nach einem der vorhergehenden Ansprüche, mit folgenden Schritten:
a. Sieben und anschließendes Mischen von Trimetazidindihydrochlorid, Polyvinylpyrrolidon und Calciumhydrogenphosphat-Dihydrat in einem Schnellmischer mit hoher Scherwirkung,
b. Granulieren des obigen Gemisches mit einer ausreichenden Menge Wasser,
c. Sieben des gebildeten feuchten Granulats, anschließendes Trocknen und Zurücksieben des trockenen Granulats,
d. Zugeben von Polyethylenoxid und kolloidalem Siliziumdioxid in das erhaltene Granulat und Mischen dieses Gemisches,
e. Sieben von Magnesiumstearat und Einmischen dieses in das erhaltene Gemisch zum Erhalt der Phase mit verzögerter Wirkstofffreisetzung,
f. Sieben und Zusammenmischen von Trimetazidindihydrochlorid, Dicalciumhydrogenphosphat-Anhydrat, kolloidalem Siliziumdioxid und rotem Eisenoxid,
g. Sieben von Magnesiumstearat und Einmischen dieses in das erhaltene Gemisch zum Erhalt der Phase mit sofortiger Wirkstofffreisetzung,
h. Kompaktieren der Phase mit verzögerter Wirkstofffreisetzung und der Phase mit sofortiger Wirkstofffreisetzung zur Bildung einer Tablette mit zwei Schichten.

13. Verwendung einer pharmazeutischen Formulierung nach einem der vorhergehenden Ansprüche zur Herstellung eines Medikaments zur Verwendung bei der Behandlung von Angina pectoris, chorioretinalen Gefäßerkrankungen, Tinnitus, Schwindel und Morbus Menière.

## Revendications

1. Formulation pharmaceutique orale solide multicouche de trimétazidine ou sel pharmaceutiquement acceptable ou polymorphe de trimétazidine, **caractérisée en ce qu'**une couche de ladite formulation conférant une libération contrôlée comprend un agent conférant une libération contrôlée qui est l'oxyde de polyéthylène dans une phase granulaire externe, tandis que l'autre couche confère une libération immédiate.

2. Formulation pharmaceutique selon la revendication 1, comprenant en outre au moins un ou plusieurs excipients.

3. Formulation pharmaceutique selon l'une quelconque des revendications précédentes, comprenant de préférence au moins une couche intermédiaire.

4. Formulation pharmaceutique selon l'une quelconque des revendications précédentes, dans laquelle la quantité en poids de dichlorhydrate de trimétazidine dans ladite couche à libération immédiate n'est pas supérieure à 25 % et est de préférence 10 % en poids de la quantité totale du dichlorhydrate de trimétazidine présent dans le comprimé.

5. Formulation pharmaceutique selon l'une quelconque des revendications précédentes, dans laquelle le rapport entre le dichlorhydrate de trimétazidine dans ladite couche à libération contrôlée et l'oxyde de polyéthylène se situe entre 0,05 et 10, de préférence entre 0,1 et 5, et plus préférablement entre 0,2 et 0,8.

6. Formulation pharmaceutique selon l'une quelconque des revendications précédentes, dans laquelle ledit excipient comprend au moins un ou un mélange de liants, de diluants, de désintégrants, d'agents de glissement et de lubrifiants.

7. Formulation pharmaceutique selon la revendication 7, **caractérisée en ce que** ledit liant contient au moins un ou un mélange de polyvinylpyrrolidone (povidone), d'hydroxypropylméthylcellulose, d'hydroxypropylcellulose, de carboxyméthylcellulose, de méthylcellulose, d'éthylcellulose et d'autres dérivés de cellulose, et de gélatine ; ledit liant étant de préférence la polyvinylpyrrolidone.

8. Formulation pharmaceutique selon la revendication 7, **caractérisée en ce que** ledit diluant contient au moins un ou un mélange de lactose, d'amidon, de mannitol, d'hydrogénophosphate de calcium dihydraté, de dihydrogénophosphate de calcium anhydre, de phosphate de calcium trihydraté, de dioxyde de silicium et de glucose ; ledit diluant étant de préférence sélectionné parmi l'hydrogénophosphate de calcium dihydraté et le dihydrogénophosphate de calcium anhydre.

9. Formulation pharmaceutique selon la revendication 7, **caractérisée en ce que** ledit agent de glissement contient au moins un ou un mélange de dioxyde de silicium colloïdal, de talc, de silicate d'aluminium et de silicate de manganésium, ledit agent de glissement étant de préférence le dioxyde de silicium colloïdal.

10. Formulation pharmaceutique selon la revendication 7, dans laquelle le lubrifiant utilisé comprend du stéarate de magnésium.

11. Formulation pharmaceutique selon l'une quelconque des revendications précédentes, constituée par
a. une couche à libération immédiate
a. du dichlorhydrate de trimétazidine à raison de 0,1 à 10 % en poids,
b. de l'hydrogénophosphate dicalcique anhydre à raison de 5 à 90 % en poids,
c. de la dioxyde de silicium colloïdal à raison de 0,1 à 5 % en poids,
d. du stéarate de magnésium à raison de 0,1 à 5 % en poids,
e. de l'oxyde de fer rouge à raison de 0,01 à 5 % en poids ;
b. une couche à libération contrôlée
a. du dichlorhydrate de trimétazidine à raison de 2,5 à 50 % en poids,
b. d'oxyde de polyéthylène à raison de 2 à 90 % en poids,
c. de la polyvinylpyrrolidone à raison de 0,1 à 20 % en poids,
d. de l'hydrogénophosphate de calcium dihydraté à raison de 5 à 90 % en poids,
e. de dioxyde de silicium colloïdal à raison de 0,1 à 5 % en poids,
f. du stéarate de magnésium à raison de 0,1 à 5 % en poids.

12. Procédé de préparation d'une formulation pharmaceutique selon l'une quelconque des revendications précédentes, comprenant les étapes consistant à
a. tamiser puis mélanger le dichlorhydrate de trimétazidine, la polyvinylpyrrolidone et l'hydrogénophosphate de calcium dihydraté dans un mélangeur à haut cisaillement,
b. granuler le mélange ci-dessus avec une quantité suffisante d'eau,
c. tamiser les granules humides formés, puis les sécher et tamiser de nouveau les granules séchés,
d. ajouter l'oxyde de polyéthylène et le dioxyde de silice colloïdale dans les granules obtenus et mélanger ceux-ci,
e. tamiser le stéarate de magnésium et le mélanger dans le mélange résultant pour obtenir la phase à libération contrôlée,
f. tamiser et mélanger ensemble le chlorhydrate de trimétazidine, l' hydrogénophosphate décalcique anhydre, le dioxyde de silicium colloïdale et l'oxyde de fer rouge,
g. tamiser le stéarate de magnésium et le mélanger dans le mélange résultant pour obtenir la phase à libération immédiate,
h. compacter la phase à libération contrôlée et la phase à libération immédiate pour obtenir un comprimé bicouche.

13. Utilisation d'une formulation pharmaceutique selon l'une quelconque des revendications précédentes dans la production d'un médicament en vue d'une utilisation dans le traitement de l'angine de poitrine, des troubles vasculaires choriorétiniens, des acouphènes, du vertige et du syndrome de Ménière.
